# EUROPEAN PATENT APPLICATION

(11) **EP 2 011 535 A1**
(43) Date of publication of application: **07.01.2009**
(21) Application number: 07013292.3
(22) Date of filing: 06.07.2007
(51) Int. Cl.: A61M 15/00

(54) **Inhaler**

(71) Applicant: Boehringer Ingelheim Pharma GmbH & Co. KG, 55216 Ingelheim am Rhein (DE); Vectura Delivery Devices Limited, Chippenham, Wiltshire SN14 6FH (GB)
(72) Inventor: Von Brunn, Timo, 10245 Berlin (DE)
(74) Representative: Gesthuysen, von Rohr & Eggert

(57) **Abstract**

An inhaler (1) for delivery of a powder-form inhalation formulation from a blister strip (2) with a plurality of blister pockets (3) is proposed. The used part of the blister strip (2) is wound up and/or kept taut and/or free of loops by means of a spring (15).

## Description

The present invention relates to an inhaler according to the preamble of claim 1.

The present invention relates to an inhaler for delivery of a powder-form inhalation formulation from a blister strip with a plurality of blister pockets (also called blisters) each of which contains a dose of the inhalation formulation.

GB 2 407 042 A discloses an inhaler with a rolled-up blister strip. For or during inhalation, in each case one dose of the inhalation formulation is taken from a blister pocket and this blister pocket is thereby emptied. This takes place during inhalation when a patient breathes in in that an air stream is passed through the previously pierced blister pocket, with the result that the inhalation formulation in the blister pocket mixes with the air and is delivered in the desired manner. The empty blister pockets are released in each case and must be disposed of.

WO 2005/037353 A1 discloses a similar inhaler, wherein the part of the blister strip with used (in particular opened and/or already empty) blister pockets - this part is also abbreviated to or called "used part" in the present invention - can be stored in the inhaler. This is carried out in that the blister strip forms an endless band (a closed loop) which can be moved in a double-threaded spiral with deflection. This structure requires relatively high forces to move the blister strip on and does not allow optimum storage of the used part. Consequently, there is a need for design solutions for the optimum storage of the used part.

The object of the present invention is to provide an inhaler which makes possible an optimized storage of the used part of a band-shaped blister strip.

The above object is achieved by an inhaler according to claim 1. Advantageous embodiments are subject of the subclaims.

A first aspect of the present invention is to form the inhaler or its receiving apparatus for the blister strip such that the used part of the blister strip is wound up in the inhaler. This allows a very compact reception and storage of the used part, with the result that the total space required can be minimized. In contrast to the state of the art, relatively small forces are needed to move on and receive the blister strip, in particular for winding-up.

The used part of the blister strip is particularly preferably wound onto a drum which is driven by a spring. This allows a very simple and compact structure, wherein the used part of the blister strip is preferably always kept taut by the spring. However, this aspect to keep tant the used part by a spring means can be used independently.

But instead of the spring, another coupling element, variable in respect of the transmission ratio and/or not rigid, can also be provided in the transmission train of the receiving apparatus or its winder.

An other embodiment variant provides that the used part is wound up by elastic force and/or in drawn into a reception space. This takes place in particular with a clock spring, arranged in the reception space, which engages at the free end of the used part of the blister strip. Thus a very compact winding-up of the used part is made possible in a simple and cost-favourable manner.

According to a further, also independently realizable aspect of the present invention, the used part is always guided or kept taut and/or free of loops. Preferably, the used part is tensioned and/or wound up by an elastic force, in particular a spring force. This allows a simple construction and/or a defined guidance of the blister strip.

Preferably, the inhaler has a conveyor for the stepwise onward movement of the blister strip in order to be able to open and/or empty the blister pockets successively for the inhalation of the respective dose. According to a particularly preferred embodiment alternative, the conveyor is formed such that the blister strip can be released in steps and in each case can preferably be moved on to the next blister pocket by elastic force alone. This allows a simplification of operation, because - in particular given the purely mechanical structure of the inhaler - a complete onward movement of the blister strip from one blister pocket to the next blister pocket by a user is not necessary. Instead a release or trigger action, which can be achieved for example with relatively less force and a movement, very short if necessary, suffices to release the blister strip, with the result that it is moved on to the next blister pocket by elastic force.

Further aspects, features, properties and advantages of the present invention result from the claims and the subsequent description of preferred embodiments, with the help of the drawing. There are shown in:
- Fig. 1: a schematic sectional representation of an inhaler as proposed, according to a first embodiment; and
- Fig. 2: a schematic sectional representation of an inhaler as proposed, according to a second embodiment.

In the Figures, the same reference numbers are used for identical or similar parts, even if a repeated description is omitted. In particular identical or corresponding advantages and properties then also result.

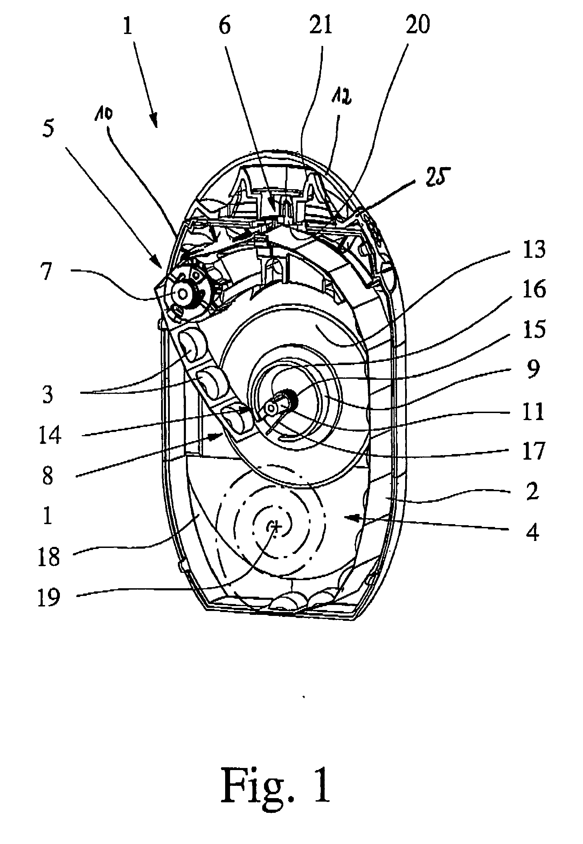

Fig. 1 shows in a schematic representation an inhaler 1 as proposed, according to a first embodiment, cut open.

The inhaler 1 serves to deliver a preferably powder-form inhalation formulation from a band-shaped blister strip 2. The blister strip 2 is preferably finite, thus it does not form an endless or closed loop. It has a large number of blister pockets 3, each of which contains directly a dose of the preferably loose inhalation formulation. To inhale and in particular upon inhalation, preferably in each case a dose of the inhalation formulation is removed from a blister pocket 3.

The inhaler 1 has a reservoir 4 for the still unused blister strip 2 with closed (sealed) blister pockets 3. In particular the blister strip 3 is rolled up or wound up in the reservoir 4. In the representation example the reservoir 4 is formed such that the blister strip 2 can be moved outwards or pulled out of the reservoir as easily as possible.

In the representation example the blister strip 2 is directly received in the reservoir 4. However, instead of this a cassette, a container, a drum or suchlike can also be fitted or inserted with the blister strip 2 into the inhaler 1 or the reservoir 4.

The inhaler 1 has preferably a conveyor 5 or any other suitable conveying means for the preferably stepwise onward movement of the blister strip 2, preferably by one blister pocket 3 in each case, in order to feed the blister pockets 3 successively - i.e. one at a time - to an opening and/or removal position 6 for the opening and/or emptying of the respective blister pocket 3 one at a time - i.e. to allow access to and/or to remove the respective dose of the inhalation formulation.

The inhaler 1 comprises a piercing element 20 to puncture a lid of the respective blister pocket 3 in position 6, i.e. aligned to the piercing element 20. The piercing element 20 is hollow and in fluid connection with an adjacent mouthpiece 21 of the inhaler 1.

The inhaler 1 comprises a lever-like actuator 22 pivotally mounted to a housing 10 of the inhaler 1. The actuator 25 supports the piercing element 20 and mouthpiece 21.

Fig. 1 shows the inhaler 1 with a mouthpiece cover 12 in the closed position. After opening the mouthpiece cover 12, the actuator 22 can be manually pivoted to retract the piercing element 20 from the last-pierced blister pocket and to pierce the next one.

The conveyor 5 is driven or actuated by the pivotal movement of the actuator 22 or by a pivotal mouthpiece cover 12.

In the representation example the conveyor 5 has a conveying wheel 7, which for example can engage between the blister pockets 3 and thus convey the blister strip 2 in preferably form-locking manner. The conveying apparatus 5 is preferably manually actuated. Possible design details follow in the description of the second embodiment.

The inhaler 1 has a receiving apparatus 8 to receive or store the used part of the blister strip 2.

The receiving apparatus 8 is preferably formed such that the used part can be wound up. The winding-up takes place in particular onto a drum 9 of the receiving apparatus 8.

In particular, axle 11 houses or supports the drum 9. Particularly preferably the drum 9 concentrically surrounds the axle 11.

The drum 9 may be located to a side blade or winding wheel 13 which preferably forms or supports or is connected with the axle 11. However, other design solutions are possible.

The inhaler 1 comprises a biasing and/or coupling element 14 that is associated to the drum 9 to drive the drum 9 for winding up the blister strip 2.

The biasing and/or coupling element 14 is preferably a spring 15, in particular a leg or helical spring. In the representation example the spring 15 is in particular formed as a spiral spring with at least one leg, preferably two legs 16, 17.

In the representation example the spring 15 is arranged with its coils preferably concentric to the rotation axis or winding axle 11.

In the representation example, the spring 15 can be coupled at one end rotation-resistant or rotatable to only a limited extent to the winding axle 11, preferably via the leg 16, in particular so that the winding axle 11 entrains or co-rotates the spring 15. Preferably the leg 16 engages for this purpose in a corresponding recess, such as an axial groove.

The other end of the spring 15 or the other leg 17 of the spring 15 is coupled to the drum 9 in the representation example. In particular the leg 17 runs substantially radially and/or engages in a corresponding recess of the drum 9 and/or is rotation-resistant or rotatable to only a limited extent to the drum 9. The drum 9 is preferably rotatably housed or supported on the winding axle 11.

In the delivered or unused state of the inhaler 1, the blister strip 2 is preferably already guided through the conveyor 5 and connected at its free end to the drum 9. This ensures secure winding. However, here too other design solutions are possible, for example such that the receiving apparatus 8 or drum 9 automatically grips the blister strip 2 or its free end and then winds up if the blister strip 2 is accordingly fed from the conveyor 5.

The spring 15 biases the drum 9 in winding-up direction and drives the drum 9 so that the used part of the blister strip 2 is wound up onto the drum 9, in particular stepwise each time the conveyor 5 moves the blister strip 4 onward.

In particular the spring 15 ensures that the unused part of the blister strip 2 is always kept or guided taut in the inhaler 1. Particularly preferably an undesired formation of loops or suchlike can thus be prevented. In the representation example the unused part of the blister strip 2 can thus be tensioned or drawn by the receiving apparatus 8.

As already said, in principle any suitable spring 15, such as a helical spring, spiral spring, clock spring or suchlike, can also be used.

In the case of the first embodiment the unused part of the blister strip 2 (represented in Fig. 1 in particular in wound-up form by dashed lines) and the receiving apparatus 8 for winding-up the used part preferably use a common housing space 18. In particular the distance of the virtual axis 19 from the unused part of the winding axis or axle 11 is smaller than the sum of the maximum winding radius of the unused part and of the maximum winding radius of the used part. This allows a particularly good use of space and accordingly a particularly compact structure of the inhaler 1 similar as in video cassettes. However, in principle a more distant arrangement of the two winding spaces is also possible.

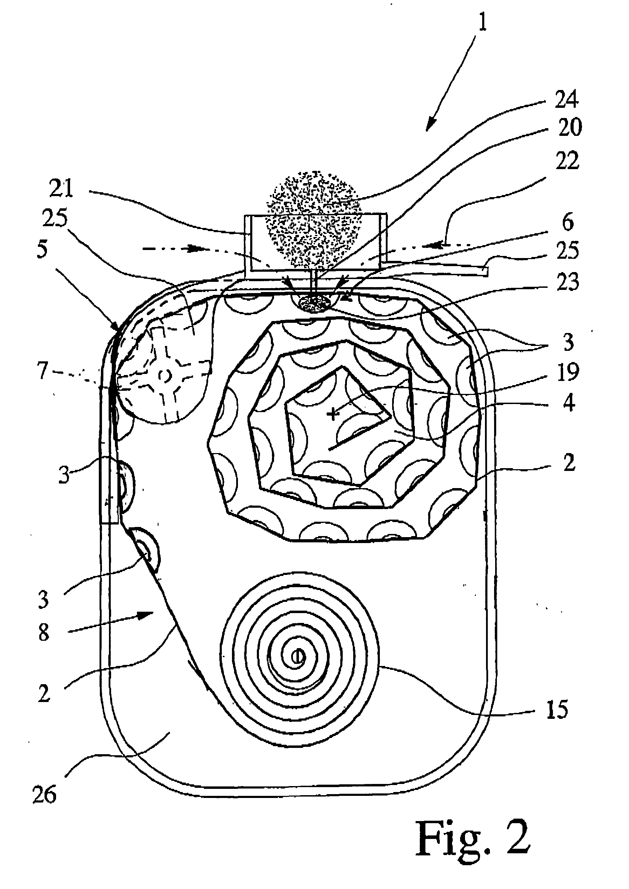

A second embodiment of the inhaler 1 as proposed is described in more detail below with the help of the schematic section of Fig. 2. To avoid repetition only substantial differences between the second embodiment and the first embodiment are explained. The previous statements and explanations relating to the first embodiment and generally to the present invention thus apply accordingly or supplementarily.

As already mentioned, the piercing element 20 allows the blister pockets 3 to be opened and/or emptied one at a time. The respective blister pocket 3 is opened from outside by the piercing element 20 which punctures a lid of the respective blister pocket 3 from outside. The piercing element 20 is formed preferably hollow and produces or allows a fluid connection to an adjacent mouthpiece 21 of the inhaler 1.

During or for inhalation a user, not represented, places the mouthpiece 21 in his mouth and breathes in. The respectively opened blister pocket 3, preferably into which the piercing element 20 extends, is thereby emptied by sucking in. An air stream 22 of ambient air is sucked in and passed through the opened blister pocket 3 such that the loose powder 23, which is shown in Fig. 2, forming the inhalation formulation is delivered with the sucked-in ambient air as an aerosol cloud 24 via the mouthpiece 21. This situation is schematically represented in Fig. 2.

The actuator 25 may serve to actuate or drive the conveyor 5 in addition to the piercing element 20.

In the case of the second embodiment, just as in the first embodiment, the piercing element 20 and the mouthpiece 21 are attached to the actuator 25.

When the actuator 25 swivels from the position shown in Fig. 2 (in particular about the axis of the conveying wheel 7 and anti-clockwise in the representation according to Fig. 2), the piercing element 20 is withdrawn from the last-pierced blister pocket 3. Simultaneously the swivelling of the actuator 25 brings about a further rotation of the conveying wheel 7, which is accordingly coupled to the actuator 25, in order to move the blister strip 2 onwards. In particular the blister strip 2 is thus conveyed onwards by one blister pocket 3. The conveying wheel 7 is preferably provided with a freewheel and a corresponding rotation lock so that upon the to-and-fro swivelling and preferably also during an incomplete swivelling of the actuator in the desired manner the conveying wheel 7 can be rotated only in one direction and in particular only in the desired steps.

When the actuator 25 swivels back into the position shown in Fig. 2 the next blister pocket 3 of the blister strip 2 is pierced by the piercing element 20 and thereby opened. Thus the inhaler 1 is ready again for the next inhalation.

The conveyor 5 or conveying wheel 7 is preferably arranged between the reservoir 4 and the receiving apparatus 8, in particular between the piercing element 20 and a reception space 26 of the receiving apparatus 8, thus after the emptying of the blister pockets 3.

In the case of the second embodiment the inhaler 1 or the receiving apparatus 8 is formed such that the used part of the blister strip 2 is preferably wound up by elastic force alone and/or is drawn into the reception space 26. For this purpose the spring 15, in particular in the form of a clock spring is provided which is fitted tensioned and is represented only schematically in Fig. 2.

In the representation example the spring 15 is arranged in the reception space 26. It preferably engages at the free end of the used part of the blister strip 2. For example the spring 15 is suspended or hooked in the end area of the blister strip 2.

In particular the spring 15 forms a winder or the receiving apparatus 8.

The further the blister strip 2 is conveyed forward by the conveyor 5, the longer becomes the (used) part of the blister strip 2 extending into the reception space 26, which is then wound up accordingly by the spring 15. In the representation example the spring 15 immediately winds up the used part. However, other design solutions are also possible which have about the same or a similar effect. As already described, the spring 15 can also wind the used part up onto the rotatable drum 9.

According to a particularly preferred development, the tensioning or pulling force of the spring 15, thus the elastic force, is large enough to move the blister strip 2 forwards and also pull it out from the reservoir 4, thus also optionally wind it up depending on the design. In this case the conveyor 5 is then preferably formed such that the blister strip 2 can be released stepwise and in each case can preferably be moved on to the next blister pocket 3 by the elastic force alone. This makes possible a particularly simple operation and handling of the inhaler 1, as only an unlocking or release, for example by actuating a key, not represented, by a user, not represented, is then necessary. Thus in particular an incomplete forward conveyance of the blister strip 2 from one blister pocket 3 to the next blister pocket 3 can be ruled out in the event of incorrect operation.

In the case of the second embodiment the spring 15 or the receiving apparatus 8 can also form the conveyor 5.

In both embodiments, the receiving apparatus 8 or winder can if necessary also form the conveyor 5 or replace it or vice versa. This simplifies the structure of the inhaler 1.

Particular advantages of the invention are that the blister strip 2 is wound up inside the inhaler 1, that there is preferably a direct or geared transmission of the rotary movement or conveying movement to the receiving apparatus 8 or for winding, that the inhaler 1 is resistant to environmental influences, that the spring 15 is particularly preferably used as drive and/or that the cost of assembly is only slightly increased compared with inhalers 1 known from the state of the art.

### List of reference numbers:

- 1: inhaler
- 2: blister strip
- 3: blister pocket
- 4: reservoir
- 5: conveyor
- 6: opening and/or removal position
- 7: conveying wheel
- 8: receiving apparatus
- 9: drum
- 10: housing
- 11: winding axle
- 12: mouthpiece cover
- 13: winding wheel
- 14: coupling element
- 15: spring
- 16: leg
- 17: leg
- 18: housing space
- 19: virtual axis
- 20: piercing element
- 21: mouthpiece
- 22: air stream
- 23: powder
- 24: aerosol cloud
- 25: actuator

## Claims

1. Inhaler (1) for delivery of an inhalation formulation from a preferably band-shaped blister strip (2) with a plurality of blister pockets (3) each of which contains one dose of the inhalation formulation, comprising:
a conveyor (5) for stepwise onward movement of the blister strip (2), and
a receiving apparatus (8) to receive a used part of the blister strip (2),
**characterized in**
the receiving apparatus (8) is formed such that the used part can be wound up, and/or
that the used part is always guided or kept taut and/or free of loops.

2. Inhaler according to claim 1, **characterized in that** the receiving apparatus (8) has a drum (9) onto which the used part can be wound.

3. Inhaler according to claim 1 or 2, **characterized in that** a spring (15) pretensions the used part in winding direction.

4. Inhaler according to claims 2 and 3, **characterized in that** the spring (15) acts on or drives the drum (9).

5. Inhaler according to claim 3 or 4, **characterized in that** the spring (15) is fitted pre-tensioned.

6. Inhaler according to one of claims 3 to 5, **characterized in that** the spring (15) is a leg spring, clock spring or spiral spring.

7. Inhaler according to one of the previous claims, **characterized in that** the receiving apparatus (8) forms the conveyor (5) for the onward movement of the blister strip (2).

8. Inhaler according to one of the previous claims, **characterized in that** the used part can be tensioned and/or wound up by an elastic force, in particular a spring force.

9. Inhaler according to one of the previous claims, **characterized in that** the used part can be tensioned or drawn by the receiving apparatus (8).

10. Inhaler according to one of the previous claims, **characterized in that** the conveyor (5) is formed such that the blister strip (2) can be released stepwise and in each case can preferably be moved on stepwise by an elastic force alone.

11. Inhaler according to one of the previous claims, **characterized in that** the inhaler (1) is designed such that by breathing in during inhalation an air stream (22) of ambient air can be sucked in to discharge the respective dose from an opened blister pocket (3) and to deliver it with the ambient air as an aerosol cloud (24).

12. Inhaler according to one of the previous claims, **characterized in that** the inhalation formulation is in powder form and each of the blister pockets (3) contains one dose of the inhalation formulation in loose form.

13. Inhaler according to one of the previous claims, **characterized in that** the inhaler (1) is portable and/or hand-held.

14. Inhaler according to one of the previous claims, **characterized in that** the inhaler (1) only works mechanically.

15. Inhaler according to one of the previous claims, **characterized in that** the inhaler (1) is preferably only manually actuated.
